(19) Europäisches Patentamt
European Patent Office
Office européen des brevets

(11) Numéro de publication: **0 179 006 B1**

(12) **FASCICULE DE BREVET EUROPEEN**

(45) Date de publication de fascicule du brevet:
**04.12.91**

(51) Int. Cl.⁵: **A61K 35/16**, C12P 21/00

(21) Numéro de dépôt: **85430031.6**

(22) Date de dépôt: **17.09.85**

Le dossier contient des informations techniques présentées postérieurement au dépôt de la demande et ne figurant pas dans le présent fascicule.

(54) **Procédé d'obtention du complexe FVIII/vWF à usage thérapeutique et produits en résultant.**

(30) Priorité: **18.09.84 FR 8414480**

(43) Date de publication de la demande:
**23.04.86 Bulletin 86/17**

(45) Mention de la délivrance du brevet:
**04.12.91 Bulletin 91/49**

(84) Etats contractants désignés:
**AT BE CH DE FR GB IT LI NL SE**

(56) Documents cités:
**US-A- 4 361 509**

**BIOLOGICAL ABSTRACTS, vol. 78, no. 8, 1984, réf.no. 57028; Philadelphia, US; H.V. STEL et al.: "Characterization of 25 monoclonal antibodies to factor VIII-von Willebrand factor: Relationship between ristocetin-induced platelet aggregation and platelet adherence to subendothelium", & BLOOD 63(6): 1408-1415. 1984**

(73) Titulaire: **IMMUNO FRANCE S.A.R.L.**
**Z.I. Senia 544 8 rue des Quinze Arpents Orly**
**F-94577 Rungis Cédex(FR)**

(84) Etats contractants désignés:
**FR**

Titulaire: **IMMUNO Aktiengesellschaft für chemisch-medizinische Produkte Industriestrasse 67 A-1221 Wien(AT)**

(84) Etats contractants désignés:
**BE CH DE GB IT LI NL SE AT**

(72) Inventeur: **Bourgois, Alain**
**123 Fc, Traverse Paragon**
**F-13008 - Marseille(FR)**
Inventeur: **Delezay, Marylène Les Eaux Vives**
**Bât. D - 248 boulevard Paul-Claudel**
**F-13010 - Marseille(FR)**
Inventeur: **Fert, Vincent**
**La Maladrerie**
**F-26110 - Nyons(FR)**

CHEMICAL ABSTRACTS, vol. 103, 1985, page 376, réf.no. 35829z, Columbus, Ohio, US; P. AVNER et al.: "Monoclonal antibodies against the human factor VIII von Willebrand molecule: characterization and potential for screening of von Willebrand patients", & DEV. BIOL. STAND. 1984, 57(Monoclonal Antibodies), 69-76

CHEMICAL ABSTRACTS, vol. 96, no. 19, 10 mai 1982, page 571, réf.no. 160565y, Columbus, Ohio, US; D.N. FASS et al.:"Monoclonal antibodies to porcine factor VIII coagulant and their use in the isolation of active coagulant protein", & BLOOD 1982, 59(3), 594-600

(74) Mandataire: Kolb, Helga, Dr. Dipl.-Chem. et al Hoffmann, Eitle & Partner Patentanwälte Arabellastrasse 4
W-8000 München 81(DE)

## Description

La présente invention concerne l'obtention du complexe FVIII/vWF à usage thérapeutique possédant à la fois l'activité anti-hémophilique A et l'activité Willebrand, ainsi qu'un haut degré de pureté.

Plusieurs procédés de purification du facteur anti-hémophilique A en vue d'une utilisation thérapeutique de ce facteur ont déjà été décrits : ils font appel à des méthodes diverses telles que cryoprécipitation, précipitation sélective, adsorption et élution fractionnée et chromatographie d'échanges d'ions.

On sait que l'activité antihémophilique A est portée par le facteur VIII (désigné par FVIII ou VIII:C) qui, à l'état physiologique, est associé au facteur Willebrand (désigné par vWF ou VIIIR:Ag) sous forme de complexe FVIII/vWF.

Des anticorps monoclonaux dirigés en particulier contre la partie Willebrand (VIIIR:Ag) ont déjà été produits (B. Sola, P. Avner, Y. Sultan, C. Jeanneau et P. Maisonneuve, P.N.A.S. 1982 vol. 79 p. 183-187, P. Avner, D. Arnaud, Y. Sultan, P. Maisonneuve et C. Jeanneau, Dev. Biol. Stand. 1984 vol. 57 p. 69-76, H.V. Stel, K.S. Sakariassen, B.J. Scholte, E.C. Veerman, T.H. van der Kwast, P.G. de Groot, J.J. Sixma et J.A. van Mouvik, Blood 1984 vol. 63 p. 1408-1415).

Les principales formes de facteur antihémophilique A à usage thérapeutique qui sont les plus généralement connues et commercialisées comprennent :

- soit le cryoprécipité qui est obtenu par décongélation du plasma sanguin frais à 4°C ;
- soit le concentré qui est obtenu à partir du cryoprécipité après deux étapes de précipitation au polyéthèneglycol.

Au sujet de ces deux formes, on notera que la concentration du cryoprécipité (lequel est très contaminé en fibrinogène) est de 5 à 10 unités internationales (U.I.) par ml, tandis que l'on peut obtenir, pour le concentré, un taux de 25 U.I. par ml.

On remarquera cependant, en ce qui concerne le concentré, qu'au cours de sa préparation, une grande partie du facteur Willebrand a été perdue, si bien que le concentré ne peut pas être efficacement utilisé pour suppléer la déficience des sujets atteints de maladie de Willebrand.

En outre, bien qu'il se présente sous une forme plus pure que le cryoprécipité, le concentré possède l'inconvénient d'être encore fortement contaminé : le complexe constitue moins de 10 % de l'ensemble des protéines dudit concentré.

Des solutions originales permettant d'obtenir un facteur VIII de haute pureté ont déjà été proposées. Une de ces solutions consiste à effectuer une gel-filtration sur Sepharose® CL2B, ce qui permet d'obtenir une fraction enrichie avec néanmoins un rapport-activité Willebrand sur activité coagulante - qui passe de 1 dans le plasma à 2 dans la fraction enrichie (B. Sola, P. Avner, Y. Sultan, C. Jeanneau et P. Maisonneuve, P.N.A.S. 1982 vol. 79 p. 183-187). Les autres solutions sont, en général, fondées sur le principe qui consiste à fixer le complexe FVIII/vWF (d'origine sanguine) sur un support solide, à laver les molécules contaminantes, puis à éluer le facteur VIII coagulant (FVIII:C) sous forme isolée, par dissociation du complexe à l'aide d'un tampon salin à haute force ionique (NaCl 1M ou CaCl2 0,25 à 0,5M). Le complexe de départ est habituellement fixé sur ledit support solide, soit à l'aide d'anticorps polyclonaux (E.G.D. Tuddenham et al. Journal of Laboratory Clinical Medicine (1979) vol. 93, page 10), soit à l'aide d'anticorps monoclonaux (T.S. Zimmermann et C.A. Fulcher, United State Patent (1982) n° 4.361.509)) couplés à des billes de gel de "Sépharose", soit à l'aide d'une résine échangeuse d'ions appropriée comme l'aminohexyl-Sépharose® (D.E.G. Austen, British Journal of Haematology (1979) vol. 43, p. 669)).

On fera cependant observer que, lors de l'élution, la liaison entre le facteur Willebrand et la phase solide n'est pas rompue et que, de ce fait, le produit obtenu par ces différents procédés consiste en facteur VIII coagulant (FVIII:C) isolé ne contenant donc pas ledit facteur Willebrand (vWF ou VIIIR:Ag), facteur qui est nécessaire aux malades Willebrand et qu'en outre, ce facteur VIII ainsi isolé n'est pas, sous sa forme physiologique (qui est normalement le complexe FVIII/vWF) où le facteur Willebrand sert à la fois de transporteur et de protecteur dudit facteur VIII. En effet, les tampons salins d'élution utilisés sont généralement incapables de dissocier une liaison antigène-anticorps. Réciproquement, la Demanderesse a constaté que les tampons classiques de dissociation des liaisons antigène-anticorps (tampons acides ou chaotropiques) détruisent l'activité coagulante du facteur VIII. D.N. Fass, G.J. Knutson, and J.A. Katzman (Blood 1982 vol. 59 p. 594-600) ont néanmoins décrit un tampon à la fois capable de dissocier une liaison antigène-anticorps et de conserver une activité coagulante. Toutefois, ce procédé n'a pas été utilisé pour la fixation et l'élution de complexe FVIII/vWF mais du VIII:C déjà dissocié, et, en outre, ce tampon (50 % éthylène-glycol) nécessite, à l'échelle industrielle, des opérations délicates pour l'élimination de l'éthylène-glycol entraînant des pertes considérables de produit.

La présente invention se propose par conséquent :

- de fournir un produit à usage thérapeutique ayant non seulement l'activité antihémophilique A mais encore l'activité Willebrand, contrairement au FVIII:C isolé qui ne possède pas cette dernière activité et au concentré dont l'activité Willebrand est insuffisante ;
- de fournir donc un tel complexe FVIII/vWF à usage thérapeutique ayant les activités ci-dessus mais présentant de plus une pureté beaucoup plus élevée que celle du cryoprécipité et du concentré ;
- de fournir au surplus un tel complexe FVIII/vWF à usage thérapeutique de haute pureté présentant une concentration en facteur VIII plus élevée que celle du cryopréci-té et du concentré ;
- de fournir ainsi une molécule physiologique dans laquelle, contrairement au FVIII:C isolé, le facteur VIII est associé au facteur Willebrand qui lui sert de transporteur et de protecteur ;
- de fournir aussi un procédé d'obtention du complexe FVIII/vWF ayant les caractéristiques définies ci-dessus avec un rendement plus élevé que celui du concentré, ce procédé étant en outre directement applicable au plasma humain.

Selon l'invention, le procédé en question est fondé sur le principe bien connu de l'immunopurification (Immunoadsorbents in protein purification E. Ruoslahti Ed. Sc. J. Immunol. 1976 Supplement N° 3, pages 1 à 86 - Characterisation of a monoclonal antibody to human interferon and its use in affinity chromatography, Stenman et al. J. et Immol. Meth. (1981) 46 p. 337-345).

Lorsque l'on applique ce principe dans le cas particulier du facteur VIII, on se rend compte que ce dernier se comporte d'une façon qui lut est propre, à savoir qu'il perd, de façon irréversible, son activité coagulante dans les solutions d'élution qui sont habituellement utilisées en immunopurification, telles que, par exemple chlorhydrate de glycine, solution tampon acétate à pH acide, KSCN et analogues.

Or, la présente invention se propose de respecter à la fois la fonction coagulante du facteur VIII et l'intégrité du complexe FVIII/vWF et vise, par conséquent, un procédé essentiellement caractérisé par le fait qu'il consiste à fixer le complexe FVIII/vWF en milieu physiologique sur un anticorps monoclonal sélectionné et à libérer ledit complexe au moyen d'un milieu d'élution approprié n'ayant pas d'effet sur la nature et les fonctions du complexe FVIII/vWF, mais capable de dissocier les liaisons entre l'anticorps monoclonal sélectionné et le complexe FVIII/vWF, puis à recueillir ainsi ce dernier sous une forme hautement purifiée.

Suivant d'autres caractéristiques :
- L'anticorps est lié à un support solide ;
- L'anticorps monoclonal est choisi parmi ceux capables tout d'abord fixer le complexe

FVIII/vWF en milieu physiologique et de libérer ensuite ce complexe dans le milieu d'élution à un pH compris entre 8 et 10,5;
- L'anticorps répondant à la définition ci-dessus est un anticorps monoclonal provenant d'un hybridome sélectionné après une série d'hybridations de cellules de myélome de souris X63 et de splénocytes de souris BALB/C immunisées avec du complexe FVIII/vWF ;
- Le milieu d'élution est choisi parmi les solutions alcalines présentant un pH compris entre 8,5 et 10,5 ;
- La solution alcaline d'élution est choisie parmi les bases suivantes : NaOH, NH₄OH, éthanolamine, diéthanolamine, triéthanolamine et analogues.

Suivant un mode de réalisation particulier, la sélection est effectuée en trois étapes :
1. Les anticorps anti-VIIIR:Ag ont été identifiés à l'aide de facteur Willebrand (VIIIR:Ag) fixé sur une plaque de titration et d'anticorps(marqué à l'iode 125) de chèvre anti-immunoglobulines de souris.
2. Un anticorps anti-VIIIR:Ag,présentant une forte affinité en milieu physiologique et une faible affinité entre pH 8,5 et 10,5, a été sélectionné.
3. Les anticorps anti-VIIIR:Ag sélectionnés comme indiqué ci-dessus sont couplés à des particules solides du type gel et ont été testés pour leur capacité à fixer le complexe FVIII/vWF à partir de plasma, ce qui se traduit par la chute des activités anti-hémophilique A et Willebrand testées in vitro.
4. Les anticorps anti-VIIIR:Ag couplés aux particules solides et capables de fixer efficacement le complexe FVIII/vWF ont été testés pour leur aptitude à relâcher le complexe FVIII/vWF en milieu alcalin (pH 8,5 à 10,5) ; les activités antihémophiliques A et Willebrand éluées sont testées in vitro par les procédés connus.

Suivant d'autres caractéristiques, le complexe FVIII/vWF recueilli peut ête concentré par un procédé connu du type ultrafiltration ou passage sur aminohexyl-Sépharose ; le procédé peut être complété par une étape de gel-filtration et/ou une étape de lyophilisation.

L'exemple suivant est donné à titre illustratif et nullement limitatif de la présente invention :

Des cellules de splénocytes de souris BALB/C hyper-immunisées avec du complexe FVIII/vWF sont fusionnées à l'aide de polyéthylèneglycol avec des cellules de myélome de souris (souche X63). Les hybridomes produisant des anticorps anti-VIIIR:Ag sont sélectionnés et les anticorps produits sont couplés à des particules solides du type gel de Sépharose® 4B afin de former des immunoadsorbants testés :

- pour leur capacité à fixer le complexe FVIII/vWF ;
- pour ceux qui ont donné un résultat positif quant à cette capacité de fixer ce complexe, pour leur aptitude à relâcher le complexe FVIII/vWF en milieu alcalin (pH 8,5 à 10,5), les activités antihémophilique A et Willebrand fixées dans le premier test ci-dessus et éluées à la suite du second, étant testées in vitro par les procédés connus.

L'anticorps monoclonal produit par l'hybridome sélectionné come ci-dessus est alors placé en milieu protéique approprié et couplé à du gel de Sépharose® 4B (polymère de dextrane). Une colonne de un litre est ensuite remplie de gel couplé à l'anticorps.

Le colonne équilibrée en chlorure de sodium 0,1 M est chargée par 10 litres de plasma passés avec un débit de 0,5 litre par heure. La colonne est ensuite lavée par 2 litres de chlorure de sodium 0,1 M puis éluée par 2 litres de triéthanolamine 100 mM (pH 9,9). La totalité du produit actif est recueillie dans un litre d'éluant et contient environ 5000 U.I. de facteur VIII et de facteur Willebrand, soit un rendement de 50 % et une concentration de 5 U.I. par ml. Ce produit contient 100 fois moins de contaminants que le cryoprécipité et 10 fois moins que son concentré. Son activité spécifique atteint 20 U.I./mg à la fois en activité VIII coagulante et en activité Willebrand.

La teneur protéique étant très faible (100 fois moins que dans le cryoprécipité), le produit peut être concentré jusqu'à 25 fois (125 U.I. par ml) soit par ultrafiltration, soit sur colonne d'aminohexyl-Sépharose. L'utilisation de la triéthanolamine comme tampon d'élution permet la lyophilisation directe du produit élué. Cette facilité est également obtenue si l'on fait appel à d'autres bases volatiles (NH4OH, éthanolamine diéthanolamine ou analogues).

La colonne rééquilibrée en chlorure de sodium 0,1 M a pu être réutilisée comme décrit ci-dessus avec les mêmes résultats.

Lorsqu'on a utilisé dans les opérations décrites ci-dessus du Séphacryl® (polymère de dextrane et d'acrylamide) à la place du Sépharose®, des concentrations de 16 U.I. par ml ont été obtenues. On peut de la même façon utiliser d'autres gels analogues comme le Trisacryl (polymère de tris et d'acrylamide).

Le produit résultant de la mise en oeuvre du procédé convient particulièrement pour restaurer les activités déficientes chez les hémophiles A et les malades Willebrand par injection intraveineuse sous faible volume (de l'ordre de 4 ml pour 500 U.I.).

Il va de soi que la présente invention n'a été décrite qu'à titre purement explicatif et nullement limitatif et que toute modification utile pourra y être apportée, au niveau des équivalences, sans sortir de son cadre. C'est ainsi, en particulier, que le procédé selon l'invention peut être appliqué à la purification de complexe FVIII/vWF à partir de cryoprécipité ou de tout autre milieu le contenant.

## Revendications

### Revendications pour les Etats contractants suivants: BE CH DE FR GB IT LI NL SE

1. Procédé d'obtention de complexes FVIII/vWF à usage thérapeutique possédant à la fois l'activité hémophilique A et l'activité Willebrand, ainsi qu'un haut degré de pureté, procédé essentiellement caractérisé par le fait qu'il consiste à fixer le complexe FVIII/vWF en milieu physiologique sur un anticorps monoclonal qui relâche ce complexe entre pH 8,5 et 10,5, et à libérer ledit complexe au moyen d'un milieu d'élution de pH 8,5 à 10,5 n'ayant pas d'effet sur les fonctions du complexe FVIII/vWF, mais dissociant les liaisons entre l'anticorps monoclonal sélectionné et le complexe FVIII/vWF, puis à recueillir ce dernier sous une forme hautement purifiée d'environ 20 U.I. par mg à la fois en activité VIII coagulante et en activité Willebrand.

2. Procédé selon la revendication 1, caractérisé par le fait que l'anticorps est lié à un support solide.

3. Procédé selon la revendication 1 ou 2, caractérisé par le fait que le même anticorps monoclonal est utilisé dans plusieurs cycles.

4. Procédé selon l'une quelconque des revendications 1 à 3, caractérisé par le fait que l'anticorps monoclonal provient d'un hybridome sélectionné après une série d'hybridations de cellules de myélome de souris X63 et de splénocytes de souris BALB/C immunisées avec le complexe FVIII/vWF.

5. Procédé selon la revendication 1, caractérisé par le fait que les bases entrant dans la composition du milieu d'élution sont de type volatil choisi dans le groupe comprenant NH4OH, éthanolamine, diéthanolamine, triéthanolamine.

6. Procédé selon l'une quelconque des revendications 1 à 5, caractérisé par le fait qu'après avoir recueilli le complexe FVIII/vWF hautement purifié, celui-ci est soumis à une opération de lyophilisation.

7. Procédé selon l'une quelconque des revendications 1 à 6, caractérisé par le fait qu'il comprend en outre une étape de concentration par ultra-filtration ou sur aminohexyl-Sépharose®.

8. Procédé selon l'une quelconque des revendications 1 à 7, caractérisé par le fait qu'il comprend en outre une étape de gel-filtration.

9. A titre de produit industriel nouveau convenant pour la mise en oeuvre du procédé selon l'une quelconque des revendications 1 à 8, un immunoadsorbant constitué d'un anticorps monoclonal anti-VIII R:Ag fixé sur un support solide, cet anticorps possédant une affinité au VIII R:Ag, forte en milieu physiologique et faible à pH compris entre 8,5 et 10,5.

10. Immunoadsorbant selon la revendication 9, caractérisé par le fait que ledit corps solide est un gel.

11. Immunoadsorbant selon la revendication 10, caractérisé par le fait que l'anticorps monoclonal est mis en présence d'un milieu protéique approprié.

12. Produit à usage thérapeutique ayant non seulement l'activité antihémophilique A mais encore l'activité Willebrand, caractérisé par le fait qu'il consiste en un complexe FVIII/vWF de haute pureté d'environ 20 U.I./mg à la fois en activité VIII coagulante et en activité Willebrand.

**Revendications pour l'Etat contractant suivant: AT**

1. Procédé d'obtention de complexes FVIII/vWF à usage thérapeutique possédant à la fois l'activité hémophilique A et l'activité Willebrand, ainsi qu'un haut degré de pureté, procédé essentiellement caractérisé par le fait qu'il consiste à fixer le complexe FVIII/vWF en milieu physiologique sur un anticorps monoclonal qui relâche ce complexe entre pH 8,5 et 10,5, et à libérer ledit complexe au moyen d'un milieu d'élution de pH 8,5 à 10,5 n'ayant pas d'effet sur les fonctions du complexe FVIII/vWF, mais dissociant les liaisons entre l'anticorps monoclonal sélectionné et le complexe FVIII/vWF, puis à recueillir ce dernier sous une forme hautement purifiée d'environ 20 U.I. par mg à la fois en activité VIII coagulante et en activité Willebrand.

2. Procédé selon la revendication 1, caractérisé par le fait que l'anticorps est lié à un support solide.

3. Procédé selon la revendication 1 ou 2, caractérisé par le fait que le même anticorps monoclonal est utilisé dans plusieurs cycles.

4. Procédé selon l'une quelconque des revendications 1 à 3, caractérisé par le fait que l'anticorps monoclonal provient d'un hybridome sélectionné après une série d'hybridations de cellules de myélome de souris X63 et de splénocytes de souris BALB/C immunisées avec le complexe FVIII/vWF.

5. Procédé selon la revendication 1, caractérisé par le fait que les bases entrant dans la composition du milieu d'élution sont de type volatil choisi dans le groupe comprenant NH4OH, éthanolamine, diéthanolamine, triéthanolamine.

6. Procédé selon l'une quelconque des revendications 1 à 5, caractérisé par le fait qu'après avoir recueilli le complexe FVIII/vWF hautement purifié, celui-ci est souris à une opération de lyophilisation.

7. Procédé selon l'une quelconque des revendications 1 à 6, caractérisé par le fait qu'il comprend en outre une étape de concentration par ultra-filtration ou sur aminohexyl-Sépharose®.

8. Procédé selon l'une quelconque des revendications 1 à 7, caractérisé par le fait qu'il comprend en outre une étape de gel-filtration.

9. Procédé d'obtention d'un immunoadsorbant pour la mise en oeuvre du procédé selon l'une quelconque des revendication 1 à 8, caractérisé par fixer un anticorps moncolonal anti-VIII R:Ag sur un support solide, cet anticorps possédant une affinité au VIII R:Ag forte en milieu physiologique et faible à pH compris entre 8,5 et 10,5.

10. Procédé selon la revendication 9, caractérisé par le fait que ledit corps solide est un gel.

11. Procédé selon la revendication 10, caractérisé par le fait que l'anticorps monoclonal est mis en presence d'un milieu protéique approprié.

12. L'usage du complexe FVIII/vWF selon les revendications 1 à 8 pour preparer un médicament pour restaurer les activités déficientes chez les hémophiles A et les malades v. Willebrand.

**Claims**

**Claims for the following Contracting States: BE CH DE FR GB IT LI NL SE**

1. Method for the production of the FVIII/vWF complex for therapeutic use having both anti-haemophilic A activity and von Willebrand activity, as well as a high degree of purity, which method is essentially characterised in that it consists of fixing the FVIII/vWF complex in a physiological medium on a monoclonal antibody which slightly binds this complex between pH 8.5 and 10.5, and releasing said complex by means of an elution medium of pH 8.5 to 10.5 having no effect on the functions of the FVIII/vWF complex, but dissociating the bonds between the selected monoclonal antibody and the FVIII/vWF complex, then collecting the latter in a highly purified form of about 20 I.U. per mg both in coagulant VIII activity and in von Willebrand activity.

2. Method according to claim 1, characterised in that the antibody is bound to a solid carrier.

3. Method according to claim 1 or 2, characterised in that the same monoclonal antibody is used in several cycles.

4. Method according to any of claims 1 to 3, characterised in that the monoclonal antibody comes from a hybridoma selected after a series of hybridizations of mouse X63 myeloma cells and mouse BALB/c splenocytes from mice immunised with FVIII/vWF complex.

5. Method according to claim 1, characterised in that the bases entering into the composition of the elution medium are of the volatile type selected from among the group including $NH_4OH$, ethanolamine, diethanolamine, triethanolamine.

6. Method according to any of claims 1 to 5, characterised in that after collecting the highly purified FVIII/vWF complex, the latter is subjected to a lyophilisation operation.

7. Method according to any of claims 1 to 6, characterised in that it further comprises a step of concentration by ultra-filtration or on aminohexyl-Sepharose®.

8. Method according to any of claims 1 to 7, characterised in that it further comprises a step of gel filtration.

9. As a novel industrial product suitable for carrying out the method according to any of claims 1 to 8, an immunoadsorbent consisting of a monoclonal antibody to VIIIR:Ag fixed on a solid carrier, this antibody having an affinity for VIIIR:Ag which is strong in a physiological medium and weak at a pH between 8.5 and 10.5.

10. Immunoadsorbent according to claim 9, characterised in that said solid body is a gel.

11. Immunoadsorbent according to claim 10, characterised in that the monoclonal antibody is placed in the presence of a suitable protein medium.

12. Product for therapeutic use having not only anti-haemophilic A activity but also von Willebrand activity, characterised in that it consists of a FVIII/vWF complex of high purity of about 20 I.U./mg both in coagulant VIII activity and in von Willebrand activity.

**Claims for the following Contracting State: AT**

1. Method for the production of the FVIII/vWF complex for therapeutic use having both anti-haemophilic A activity and von Willebrand activity, as well as a high degree of purity, which method is essentially characterised in that it consists of fixing the FVIII/vWF complex in a physiological medium on a monoclonal antibody which slightly binds this complex between pH 8.5 and 10.5, and releasing said complex by means of an elution medium of pH 8.5 to 10.5 having no effect on the functions of the FVIII/vWF complex, but dissociating the bonds between the selected monoclonal antibody and the FVIII/vWF complex, then collecting the latter in a highly purified form of about 20 I.U. per mg both in coagulant VIII activity and in von Willebrand activity.

2. Method according to claim 1, characterised in that the antibody is bound to a solid carrier.

3. Method according to claim 1 or 2, characterised in that the same monoclonal antibody is used in several cycles.

4. Method according to any of claims 1 to 3, characterised in that the monoclonal antibody comes from a hybridoma selected after a series of hybridizations of mouse X63 myeloma cells and mouse BALB/c splenocytes from mice immunised with FVIII/vWF complex.

5. Method according to claim 1, characterised in that the bases entering into the composition of the elution medium are of the volatile type selected from among the group including $NH_4OH$, ethanolamine, diethanolamine, triethanolamine.

6. Method according to any of claims 1 to 5, characterised in that after collecting the highly purified FVIII/vWF complex, the latter is subjected to a lyophilisation operation.

7. Method according to any of claims 1 to 6, characterised in that it further comprises a step of concentration by ultra-filtration or on aminohexyl-Sepharose®.

8. Method according to any of claims 1 to 7, characterised in that it further comprises a step of gel filtration.

9. Method for the production of an inmunoadsorbent for carrying out the method according to any of claims 1 to 8, characterised by fixing a monoclonal antibody to VIIIR:Ag on a solid carrier, this antibody having an affinity for VIIIR:Ag which is strong in a physiological medium and weak at a pH between 8.5 and 10.5.

10. Method according to claim 9, characterised in that said solid body is a gel.

11. Method according to claim 10, characterised in that the monoclonal antibody is placed in the presence of a suitable protein medium.

12. Use of the FVIII/vWF complex according to claims 1 to 8 for the preparation of a drug for restoring deficient activities in those suffering from haemophilia A and von Willebrand's disease.

**Patentansprüche**
**Patentansprüche für folgende Vertragsstaaten:**
**BE CH DE FR GB IT LI NL SE**

1. Verfahren zur Herstellung von FVIII/vWF-Komplexen zur therapeutischen Verwendung, die gleichzeitig Hämophilie A-Aktivität und Willebrand-Aktivität besitzen, sowie einen hohen Reinheitsgrad, das im wesentlichen durch die folgenden Schritte gekennzeichnet ist:
Fixieren des VIII/vWF-Komplexes in einem physiologischen Milieu an einen monoclonalen Antikörper, der den Komplex zwischen pH 8,5 und 10,5 locker bindet, und diesen Komplex mit Hilfe eines Elutionsmilieus vom pH-Wert 8,5 bis 10,5 freisetzt, das die Funktionen des FVIII/vWF-Komplexes nicht beeinträchtigt, aber die Bindungen zwischen dem ausgewählten monoclonalen Antikörper und dem FVIII/vWF-Komplex spaltet, wonach der letztere in Form einer hohen Reinheit mit ca. 20 I.E. pro mg bei gleichzeitiger koagulierender VIII-Aktivität und Willebrand-Aktivität erhalten wird.

2. Verfahren nach Anspruch 1, dadurch gekennzeichnet, daß der Antikörper an einen festen Träger gebunden ist.

3. Verfahren nach Anspruch 1 oder 2, dadurch gekennzeichnet, daß der gleiche monoclonale Antikörper in mehreren Zyklen verwendet wird.

4. Verfahren nach einem der vorhergehenden Ansprüche 1 bis 3, dadurch gekennzeichnet, daß der monoclonale Antikörper aus einem Hybridom stammt, ausgewählt nach einer Serie von Zellhybridisierungen von Mäusemyelom X63 und Splenozyten von BALB/C-Maus, immunosiert mit dem Komplex FVIII/vWF.

5. Verfahren nach Anspruch 1, dadurch gekennzeichnet, daß die in der Zusammensetzung des Elutionsmilieus enthaltenen Basen flüchtig sind und ausgewählt sind aus der Gruppe $NH_4OH$, Ethanolamin, Diethanolamin, Triethanolamin.

6. Verfahren nach einem der Ansprüche 1 bis 5, dadurch gekennzeichnet, daß man den in hoher Reinheit erhaltenen FVIII/vWF-Komplex einer Lyophilisation unterwirft.

7. Verfahren nach einem der Ansprüche 1 bis 6, dadurch gekennzeichnet, daß es außerdem eine Stufe der Konzentration mittels Ultrafiltration oder Aminohexyl-Sepharose® umfaßt.

8. Verfahren nach einem der Ansprüche 1 bis 7, dadurch gekennzeichnet, daß es außerdem eine Stufe der Gelfiltration umfaßt.

9. Neues Immunoadsorbens, bestehend aus einem monoclonalen Anti-VIII R:Ag-Antikörper, der an einen festen Träger gebunden ist, wobei dieser Antikörper gegenüber VIII R:Ag eine starke Affinität in einem physiologischen Milieu und eine schwache bei einem pH-Wert zwischen 8,5 und 10,5 zeigt, geeignet zur Durchführung eines Verfahrens gemäß einem der Ansprüche 1 bis 8.

10. Immunoadsorbens nach Anspruch 9, dadurch gekennzeichnet, daß der feste Körper ein Gel ist.

11. Immunoadsorbens gemäß Anspruch 10, dadurch gekennzeichnet, daß der monoclonale Antikörper in Gegenwart eines geeigneten Protein-Milieus vorliegt.

12. Produkt zur therapeutischen Verwendung, das nicht nur Antihämophilie A-Aktivität, sondern gleichzeitig Willebrand-Aktivität aufweist, dadurch gekennzeichnet, daß es aus einem FVIII/vWF-Komplex hoher Reinheit mit ungefähr 20 I.E./mg gleichzeitiger koagulierender VIII-Aktivität und Willebrand-Aktivität besteht.

**Patentansprüche für folgenden Vertragsstaat: AT**

1. Verfahren zur Herstellung von FVIII/vWF-Komplexen zur therapeutischen Verwendung, die gleichzeitig Hämophilie A-Aktivität und Willebrand-Aktivität besitzen, sowie einen hohen Reinheitsgrad, das im wesentlichen durch die folgenden Schritte gekennzeichnet ist:
Fixieren des VIII/vWF-Komplexes in einem physiologischen Milieu an einen monoclonalen Antikörper, der den Komplex zwischen pH 8,5 und 10,5 locker bindet, und diesen Komplex mit Hilfe eines Elutionsmilieus vom pH-Wert 8,5 bis 10,5 frei-setzt, das die Funktionen des FVIII/vWF-Komplexes nicht beeinträchtigt, aber die Bindungen zwischen dem ausgewählten monoclonalen Antikörper und dem FVIII/vWF-Komplex spaltet, wonach der letztere in Form einer hohen Reinheit mit ca. 20 I.E. pro mg bei gleichzeitiger koagulierender VIII-Aktivität und Willebrand-Aktivität erhalten wird.

2. Verfahren nach Anspruch 1, dadurch gekennzeichnet, daß der Antikörper an einen festen Träger gebunden ist.

3. Verfahren nach Anspruch 1 oder 2, dadurch gekennzeichnet, daß der gleiche monoclonale Antikörper in mehreren Zyklen verwendet wird.

4. Verfahren nach einem der vorhergehenden Ansprüche 1 bis 3, dadurch gekennzeichnet, daß der monoclonale Antikörper aus einem Hybridom stammt, ausgewählt nach einer Serie von Zellhybridisierungen von Mäusemyelom X63 und Splenozyten von BALB/C-Maus, immunosiert mit dem Komplex FVIII/vWF.

5. Verfahren nach Anspruch 1, dadurch gekennzeichnet, daß die in der Zusammensetzung des Elutionsmilieus enthaltenen Basen flüchtig sind und ausgewählt sind aus der Gruppe $NH_4OH$, Ethanolamin, Diethanolamin, Triethanolamin.

6. Verfahren nach einem der Ansprüche 1 bis 5, dadurch gekennzeichnet, daß man den in hoher Reinheit erhaltenen FVIII/vWF-Komplex einer Lyophilisation unterwirft.

7. Verfahren nach einem der Ansprüche 1 bis 6, dadurch gekennzeichnet, daß es außerdem eine Stufe der Konzentration mittels Ultrafiltration oder Aminohexyl-Sepharose® umfaßt.

8. Verfahren nach einem der Ansprüche 1 bis 7, dadurch gekennzeichnet, daß es außerdem eine Stufe der Gelfiltration umfaßt.

9. Verfahren zur Herstellung eines Immunoadsorbens zur Durchführung eines Verfahrens gemäß einem der Ansprüche 1 bis 8, dadurch gekennzeichnet, daß man einen monoclonalen Anti-VIII R:Ag-Antikörper an einem festen Träger fixiert, wobei dieser Antikörper gegenüber VIII R:Ag in einem physiologischen Milieu eine starke Affinität, und bei einem pH-Wert zwischen 8,5 und 10,5 eine schwache Affinität besitzt.

10. Verfahren nach Anspruch 9, dadurch gekennzeichnet, daß der feste Körper ein Gel ist.

11. Verfahren nach Anspruch 10, dadurch gekennzeichnet, daß der monoclonale Antikörper in Gegenwart eines geeigneten Protein-Milieus vorliegt.

12. Verwendung eines FVIII/vWF-Komplexes gemäß einem der Ansprüche 1 bis 8 zur Herstellung eines Medikaments zur Wiederherstellung der fehlenden Aktivitäten bei Hämophilie A- und Willebrand-Kranken.